# EUROPEAN PATENT SPECIFICATION

(11) **EP 1 844 001 B1**
(45) Date of publication and mention of the grant of the patent: **10.06.2009**
(21) Application number: 06703570.9
(22) Date of filing: 24.01.2006
(51) Int. Cl.: C07C 233/00, C07D 213/74, C07D 211/14, A61K 31/185, A61P 11/00

(54) **METABOLITES OF 2-ARYLPROPIONIC ACID DERIVATIVES AND PHARMACEUTICAL COMPOSITIONS CONTAINING THEM**
METABOLITEN AUS 2-ARYLPROPIONSÄURE-DERIVATEN UND PHARMAZEUTISCHE ZUSAMMENSETZUNGEN DAMIT
METABOLITES DE DERIVES DE L'ACIDE 2-ARYLPROPIONIQUE C ET PREPARATIONS PHARMACEUTIQUES LES CONTENANT

(30) Priority: 25.01.2005 EP 05001399
(43) Date of publication of application: 17.10.2007
(73) Proprietor: DOMPE' pha.r.ma S.p.A., 67100 L'Aquila (IT)
(72) Inventor: BERTINI, Riccardo Dompé pha.r.ma. S.p.A., I-67100 L'aquila (IT); BIZZARRI, Cinzia Dompé pha.r.ma. S.p.A., I-67100 L'aquila (IT); MOSCA, Marco Dompé pha.r.ma. S.p.A., I-67100 L'aquila (IT); ALLEGRETTI, Marcello Dompé pha.r.ma. S.p.A., I-67100 L'aquila (IT); COLOTTA, Francesco Dompé pha.r.ma. S.p.A., I-67100 L'Aquila (IT)
(74) Representative: Perani, Aurelio
(86) International application number: PCT/EP2006/050407
(87) International publication number: WO 2006/079624

(56) References cited:
- WO-A-00/24710
- WO-A-01/58852
- WO-A-02/068377
- AURELI L ET AL: "Predicting human serum albumin affinity of Interleukin-8 (CXCL8) inhibitors by 3D-QSPR approach" JOURNAL OF MEDICINAL CHEMISTRY, vol. 48, 3 August 2005 (2005-08-03), pages 2469-2479, XP002380701

## Description

### Brief description of the invention

The present invention relates to novel active metabolites of 2-(R)-arylpropionic acid derivatives and to pharmaceutical compositions containing them, which are used as inhibitors of the chemotaxis of polymorphonucleate and mononucleate cells, particularly in the treatment of neutrophils-dependent pathologies.

### State of the art

Particular blood cells (macrophages, granulocytes, neutrophils, polymorphonucleated) respond to a chemical stimulus (when stimulated by substances called chemokines) by migrating along the concentration gradient of the stimulating agent, through a process called chemotaxis. The main known stimulating agents or chemokines are represented by the breakdown products of complement C5a, some N-formyl peptides generated from lysis of the bacterial surface or peptides of synthetic origin, such as formyl-methionyl-leucyl-phenylalanine (f-MLP) and mainly by a variety of cytokines, including Interleukin-8 (IL-8, also referred to as CXCL8). Interleukin-8 is an endogenous chemotactic factor produced by most nucleated cells such as fibroblasts and macrophages.

In some pathological conditions, marked by exacerbated recruitment of neutrophils, a more severe tissue damage at the site is associated with the infiltration of neutrophilic cells. Recently, the role of neutrophilic activation in the determination of damage associated with post ischemia reperfusion and pulmonary hyperoxia was widely demonstrated.

The biological activity of IL-8 is mediated by the interaction of the interleukin with CXCR1 and CXCR2 membrane receptors which belong to the family of seven transmembrane receptors, expressed on the surface of human neutrophils and of certain types of T-cells (L. Xu et al., J. Leukocyte Biol., 57, 335, 1995). Selective ligand are known which can distinguish between CXCR1 and CXCR2: GRO-α is an example of a CXCR2 selective chemotactic factor.

Potential pathogenic role of IL-8 in pulmonary diseases (lung injury, acute respiratory distress syndrome, asthma, chronic lung inflammation, and cystic fibrosis) and, specifically, in the pathogenesis of COPD (chronic obstructive pulmonary disease) through the CXCR2 receptor pathway has been widely described (D. WP Hay and H.M. Sarau., Current Opinion in Pharmacology 2001, 1:242-247). In response to immunologic and infective events, activation of the complement system mediates amplification of inflammatory response both via direct membrane action and via release of a series of peptide fragments, generally known as anaphylatoxins, generated by enzymatic cleavage of the C3, C4 and C5 complement fractions. These peptides include C3a, C4a, both made of 77 aminoacids; in turn, C5 convertase cleaves the C5 complement fraction to give the glycoprotein C5a of 74 aminoacids.

The C5a peptide fragment of the complement has been defined as the "complete" pro-inflammatory mediator. On the contrary, other inflammatory mediators such as selected cytokines (IL-8, MCP-1 and RANTES, for example) are highly selective towards self attracted cells, while histamine and bradykinin are only weak chemotactic agents. Convincing evidences support the involvement of C5a, in vivo, in several pathological conditions including ischemia/reperfusion, autoimmune dermatitis, membrane-proliferative idiopathic glomerulonephritis, airway irresponsiveness and chronic inflammatory diseases, ARDS and CODP, Alzheimer's disease, juvenile rheumatoid arthritis (N.P. Gerard, Ann. Rev. Immunol., 12, 755, 1994).

In view of the neuro-inflammatory potential of C5a/C5a-desArg generated by both local complement production and amyloid activation joined with astrocyte and microglia chemotaxis and activation directly induced by C5a, complement inhibitors have been proposed for the treatment of neurological diseases such as Alzheimer's disease (McGeer & McGeer P.L., Drugs, 55, 738, 1998).

Therefore, the control of the local synthesis of complement fractions is considered of high therapeutic potential in the treatment of shock and in the prevention of rejection (multiple organ failure and hyperacute graft rejection) (Issekutz A.C. et al., Int. J. Immunopharmacol, 12, 1, 1990;Inagi R. et at., Immunol. Lett., 27, 49, 1991). More recently, inhibition of complement fractions has been reported to be involved in the prevention of native and transplanted kidney injuries taking account of complement involvement in the pathogenesis of both chronic interstitial and acute glomerular renal injuries. (Sheerin N.S. & Sacks S.H., Curr. Opinion Nephrol. Hypert., 7, 395, 1998). Characteristic neutrophil accumulation occurs in acute and chronic pathologic conditions, for example in the highly inflamed and therapeutically recalcitrant areas of psoriatic lesions. Neutrophils are chemotactically attracted and activated by the sinergistic action of chemokines, IL-8 and Gro-a released by the stimulated keratinocytes, and of the C5a/C5a-desArg fraction produced via the alternative complement pathway activation (T. Terui et al., Exp. Dermatol., 9, 1, 2000). In many circustances it is, therefore, highly desirable to couple cell activation stimulated by C5a, modulation of the cytokine receptors and inhibition of chemotaxis in one single agent. We have recently described a novel class of "omega-aminoalkylamides of r-2-arylpropionic acids" as inhibitors of the chemotaxis of polymorphonucleate and mononucleate cells" (WO 02/068377). The novel class include compounds ranging from selective C5a inhibitors to dual C5a/IL-8 inhibitors.

Furthermore, novel classes of potent and selective inhibitors of IL-8 biological activities (R-2-arylpropionic acid amides and N-acylsulfonamides) have been described as effective inhibitors of IL-8 induced neutrophils chemotaxis and degranulation (WO 01/58852; WO 00/24710).

### Detailed description of the invention

We have now found a novel class of 2-(R)-arylpropionic acid derivatives as inhibitors of the chemotaxis of polymorphonucleate and mononucleate cells. In particular, compounds of the inventions thereof are potent inhibitors of IL-8 induced neutrophils chemotaxis and C5a induced neutrophils and monocytes chemotaxis with improved pharmacokinetic characteristics and pharmacological activity profile.

The present invention thus provides 2-(R)-arylpropionic acid derivative compounds of formula (I) : 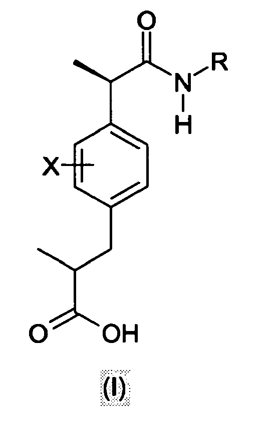 and pharmaceutically acceptable salts thereof,
wherein
X is selected from H, halogen, C₁-C₄-alkyl, C₁-C₄-alkoxy, hydroxy, cyano, nitro, amino;
R group is selected from
   - H, OH, C₁-C₅-alkyl, C₁-C₅-cycloalkyl, C₁-C₅-alkenyl, C₁-C₅-alkoxy;
   - an heteroaryl group selected from pyridine, pyrimidine, pyrrole, tiofene, furane, indole;
   - an amino acid residue consisting of straight or branched C₁-C₆-alkyl, C₁-C₆-cycloalkyl, C₁-C₆-alkenyl, C₁-C₆-phenylalkyl, substituted with one further carboxy (COOH) group;
   - a residue of formula -CH₂-CH₂-Z-(CH₂-CH₂O)nR' wherein R' is H or C₁-C₅-alkyl, n is an integer from 0 to 2 and Z is oxygen or sulfur;
   - a residue of formula -(CH₂)n-NRaRb wherein n is an integer from 0 to 5 and each Ra and Rb, which may be the same or different, are C₁-C₆-alkyl, C₁-C₆-alkenyl or, alternatively, Ra and Rb, together with the nitrogen atom to which they are bound, form a heterocycle from 3 to 7 members of formula (II) 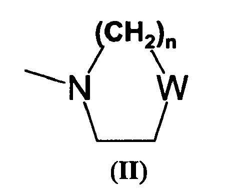
wherein W represents a single bond, O, S, N-Rc, Rc being H, C₁-C₆-alkyl or C₁-C₆-alkylphenyl, and n is an integer from 0 to 4.
   - a residue of formula SO₂Rd wherein Rd is C₁-C₆-alkyl, C₁-C₆-cycloalkyl, C₁-C₆-alkenyl.

Compounds of formula (I) are chiral compounds and the invention provides the (2R, 2"R,S) mixture and the single (2R,2"S), (2R,2'R) enantiomers.

The present invention further provides compounds of formula (I) for use as medicaments. In particular, such medicaments are inhibitors of the chemotaxis of polymorphonucleate and mononucleate cells.

Compounds of Formula (III) wherein R and X are as above defined were previously described as particularly preferred compounds among the inhibitors of the chemotaxis of polymorphonucleate and mononucleate cells reported in WO 01/58852; WO 00/24710 and WO 02/068377.

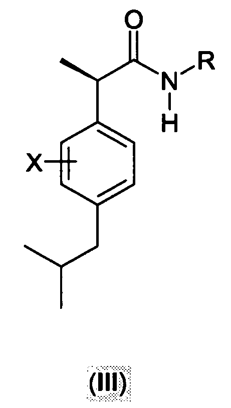

Administration "in vivo" of compounds of formula (III) contributed to elucidate the metabolic fate of the drugs. Cytochrome mediated oxidation of the isobutyl group generates the three hydroxylated isomers on the 1,2 and 3 positions of the chain; the exhaustive oxidation of the C-terminal position leads to the compounds of formula (I).

All the detected metabolites have been independently synthesized on the lab scale and tested in the chemotaxis assays.

Among the above metabolites, we have found that only compounds of Formula I are active as inhibitors of the chemotaxis of polymorphonucleate and mononucleate cells.

This finding is in contrast with the lack of biological activity of the corresponding metabolite of 2-(4-isobutylphenyl)propionic acid (ibuprofen), which is in fact, reported to be devoid of any antinflammatory activity ("Ibuprofen. A critical bibliographic review" Edited by KD Rainsford, Sheffield Hallam University 1999, pag. 42), (American Society of Health-System Pharmacists, AHFS Drug Information 2001, pag. 1918).

Compounds of Formula (I) have been shown to share significant advantageous characteristics as compared to the compounds of formula (III).

The compounds of the invention inhibit IL-8 and/or C5a induced PMN chemotaxis with an IC₅₀ comparable to the IC₅₀ of the preferred compounds of formula (III) but have been surprisingly found to be more potent inhibitors than the compounds of formula (III) in the inhibition of GRO-α induced PMN chemotaxis so indicating a specific action on the CXCR2 mediated pathway.

Furthermore, the compounds of formula (I) have a pharmacokinetic profile particularly advantageous for the therapeutic use. In fact, as compared to the compounds of formula (III), the compounds of the invention, administered "in vivo" by the i.v. route, show a longer T_{1/2} associated to a reduced clearance time.

These characteristics, associated to a lower binding to the plasmatic proteins, confer a better overall pharmacological profile to these drugs.

Preferred X group is H;
Preferred R groups are
H, OH, C₁-C₅ alkyl, C₁-C₅ alkoxy, C₁-C₂ -carboxyalkyl;
pyridine, pyrimidine;
a residue of formula -CH₂-CH₂-O-(CH₂-CH₂O)nR' wherein R' is H or C₁-C₅-alkyl, n is the integer 0 or 1;
a residue of formula -(CH₂)n-NRaRb wherein n is the integer 2 or 3, more preferably 3 and the group NRaRb is N,N-dimethylamine, N,N-diethylamine, 1-piperidyl, 4-morpholyl, 1-pyrrolidyl, 1-piperazinyl, 1-(4-methyl)piperazinyl;
a residue of formula SO₂Rd wherein Rd is C₁-C₂-alkyl.

Preferred compounds of formula (I) are the single (R) and (S) enantiomers thereof.

Particularly preferred compounds of the invention are:
(2R) (2" R,S) 2-[4'-(2"-carboxyprop-1-yl)phenyl]propionyl methanesulfonamide
(2R) (2" R,S) 2-[4'-(2"-carboxyprop-1-yl)phenyl]propionamide
(2R) (2" R,S) 2-[4'-(2"-carboxyprop-1-yl)phenyl]-N-(4"'-pyridyl)propionamide
(2R) (2" R,S) 2-[4'-(2"-carboxyprop-1-yl)phenyl]-N-(2"'-carboxymethyl) propionamide
(2R) (2" R,S) 2-[4'-(2"-carboxyprop-1-yl)phenyl]-N-(2"'-methoxyethyl)propionamide
(2R)(2"R,S)2-[4'-(2"-carboxyprop-1-yl)phenyl]-N-[3"-N'-piperidinopropyl] propionamide
(2R)(2"R,S)2-[4'-(2"-carboxyprop-1-yl)pbenyl]-N-[3"'-N',N'-dimetbylwninopropyl] propionamide and the single R and S enantiomers thereof.

The compounds of the invention are potent and selective inhibitors of the human PMNs chemotaxis induced by IL-8. The compounds of the invention wherein R is a residue of formula -(CH₂)n-NRaRb are dual inhibitors of the C5a induced and IL-8 induced PMNs chemotaxis.

The compounds of the invention of formula (I) are generally isolated in the form of their addition salts with both organic and inorganic pharmaceutically acceptable acids or bases. Examples of such acids are selected from hydrochloric acid, sulfuric acid, phosphoric acid, metansulfonic acid, fumaric acid, citric acid. Examples of such bases are sodium hydroxide, potassium hydroxide, calcium hydroxide , (D,L)-Lysine, L-Lysine, tromethamine.

Compounds of formula (I) are obtained by treatment of corresponding 2-(4'-aryl)propionamide derivatives of formula (IV), wherein W is Br or OSO₂CF_{3,} with 2-methylacrylic acid in presence of a suitable catalyst, such as palladium, to give the coupling products of formula (V) and (VI).

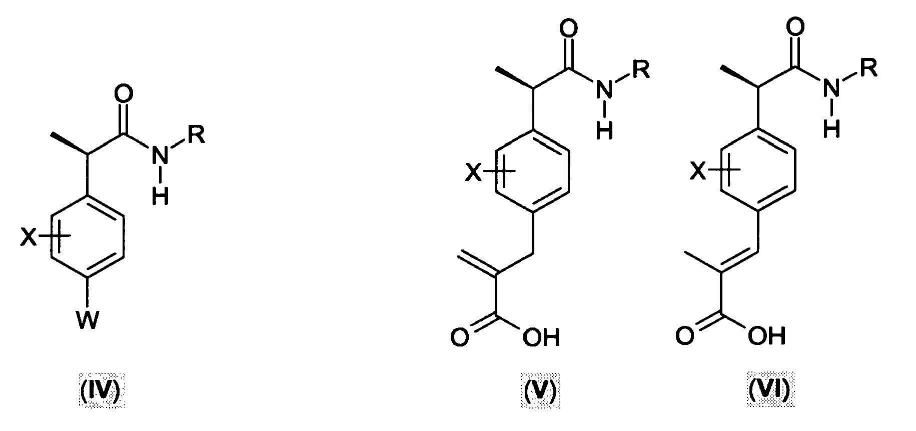

The mixture of (V) and (VI) is subsequently hydrogenated in the presence of a suitable catalyst, such as Pd/C, to give the compounds of the invention of formula (I).

According to a novel alternative synthetic pathway, compounds of formula (I), wherein R is a group as defined above, but is not OH or C₁-C₅-alkoxy or a residue SO₂Rd are obtained starting from an acylmethansulfonamide derivative of formula (I), wherein R is SO₂Rd by treatment of said derivative with two equivalents of a suitable amine of formula NH₂R, and simply heating the salt thereby obtained at a temperature of about 100-140°C, preferably under vacuum.

The compounds of the invention of formula (I) were evaluated *in vitro* for their ability to inhibit chemotaxis of polymorphonucleate leukocytes (hereinafter referred to as PMNs) and monocytes induced by the fractions of IL-8 and GRO-α and C5a and directly compared to the corresponding parent 4-isobutyl compounds of formula (III). For this purpose, in order to isolate the PMNs from heparinized human blood, taken from healthy adult volunteers, mononucleates were removed by means of sedimentation on dextran (according to the procedure disclosed by W.J. Ming et al., J. Immunol., 138, 1469, 1987) and red blood cells by a hypotonic solution. The cell vitality was calculated by exclusion with Trypan blue, whilst the ratio of the circulating polymorphonucleates was estimated on the cytocentrifugate after staining with Diff Quick.

In the IL-8 induced chemotaxis assay human recombinant IL-8 (Pepro Tech) was used as stimulating agents in the chemotaxis experiments: the lyophilized protein was dissolved in a volume of HBSS containing 0.2% bovin serum albumin (BSA) so thus to obtain a stock solution having a concentration of 10⁻⁵ M to be diluted in HBSS to a concentration of 10-⁹ M, for the chemotaxis assays.

GRO-α induced chemotaxis inhibition was evaluated in an analogous assay.

In the C5a induced chemotaxis essay the fractions hr-C5a and hrC5a-desArg (Sigma) were used as stimulating agents in chemotaxis experiments, obtaining practically identical results. Lyophilized C5a was dissolved in a volume of HBSS containing 0.2% BSA so as to obtain a stock solution having a concentration of 10⁻⁵ M, to be diluted in HBSS to a concentration of 10⁻⁹ M, for the chemotaxis assays.

In the chemotaxis experiments, the PMNs were incubated with the compounds of the invention of formula (I) for 15' at 37°C in an atmosphere containing 5% CO₂

The chemotactic activity of the C5a was evaluated on human circulating polymorphonucleates (PMNs) resuspended in HBSS at a concentration of 1.5x10⁶ PMNs per ml.

During the chemotaxis assay (according to W. Falket et al., J. Immunol. Methods, 33, 239, 1980) PVP-free filters with a porosity of 5 µm and microchambers suitable for replication were used.

The compounds of the invention in formula (I) were evaluated at a concentration ranging between 10⁻⁶ and 10⁻¹⁰ M; for this purpose they were added, at the same concentration, both to the lower pores and the upper pores of the microchamber. Evaluation of the ability of the compounds of the invention of formula (I) to inhibit the chemotaxis of human monocytes was carried out according to the method disclosed by Van Damme J. et al. (Eur. J. Immunol., 19, 2367, 1989).

The compound of Example 1, (2R) (2"R,S) 2-[4'-(2"-carboxyprop-1-yl)phenyl]propionyl methanesulfonamide, shows, for example, inhibition of IL-8-induced PMN migration, evaluated as 49±9 % at a concentration 10⁻⁸M and as 66±8 % at a concentration 10⁻⁶M.

Particularly preferred compounds of the invention are:
(2R) (2"S) 2-[4'-(2"-carboxyprop-1-yl)phenyl]propionyl methanesulfonamide
(2R) (2"R) 2-[4'-(2"-carboxyprop-1-yl)phenyl]propionyl methanesulfonamide
(2R) (2"R) 2-[4'-(2"-carboxyprop-1-yl)phenyl]-N-[3"'-N'-piperidinopropyl] propionamide
(2R) (2"S) 2-[4'-(2"-carboxyprop-1-yl)phenyl]-N-[3"'-N'-piperidinopropyl] propionamide.

The compounds of the invention, as compared to the described 2-(4-isobutylphenyl)propionamides of formula (III), show the additional property to effectively inhibit the GROα induced PMN chemotaxis; this activity allows the therapeutical use of these compounds in IL-8 related pathologies where the CXCR2 pathway is involved specifically or in conjunction with the CXCR1 signaling.

The dual inhibitors of the IL-8 and GRO-α induced biological activities are strongly preferred in view of the therapeutical applications of interest.

The compounds of formula (I), evaluated *ex vivo* in the blood *in toto* according to the procedure disclosed by Patrignani et al., in J. Pharmacol. Exper. Ther., 271, 1705, 1994, were found to be totally ineffective as inhibitors of cyclooxygenase (COX) enzymes.

In most cases, the compounds of formula (I) do not interfere with the production of PGE₂ induced in murine macrophages by lipopolysaccharides stimulation (LPS, 1 µg/mL) at a concentration ranging between 10⁻⁵ and 10⁻⁷ M. Inhibition of the production of PGE₂ which may be recorded, is mostly at the limit of statistical significance, and more often is below 15-20% of the basal value. The reduced effectiveness in the inhibition of the CO constitutes an advantage for the therapeutical application of compounds of the invention in as much as the inhibition of prostaglandin synthesis constitutes a stimulus for the macrophage cells to amplify synthesis of TNF-α (induced by LPS or hydrogen peroxide) that is an important mediator of the neutrophilic activation and stimulus for the production of the cytokine Interleukin-8.

Inhibitors of CXCR1 and CXCR2 activation find useful applications, as above detailed, particularly in treatment of chronic inflammatory pathologies (e.g. psoriasis) in which the activation of both IL-8 receptors is supposed to play a crucial pathophysiological role in the development of the disease. In fact, activation of CXCR1 is known to be essential in IL-8-mediated PMN chemotaxis (Hammond M et al, J Immunol, 155, 1428, 1995). On the other hand, activation of CXCR2 activation is supposed to be essential in IL-8-mediated epidermal cell proliferation and angiogenesis of psoriatic patients (Kulke R et al., J Invest Dermatol, 110, 90, 1998).

In addition, CXCR2 antagonists find particularly useful therapeutic applications in the management of important pulmonary diseases like chronic obstructive pulmonary disease COPD (D. WP Hay and H.M. Sarau., Current Opinion in Pharmacology 2001, 1:242-247). In view of the experimental evidence discussed above and of the role performed by Interleukin-8 (IL-8) and congenetics thereof in the processes that involve the activation and the infiltration of neutrophils, the compounds of the invention are particularly useful in the treatment of diseases such as psoriasis (R. J. Nicholoff et al., Am. J. Pathol., 138, 129, 1991), intestinal chronic inflammatory pathologies such as ulcerative colitis (Y. R. Mahida et al., Clin. Sci., 82, 273, 1992) and melanoma, chronic obstructive pulmonary disease (COPD), bullous pemphigo, rheumatoid arthritis (M. Selz et al., J. Clin. Invest., 87, 463, 1981), idiopathic fibrosis (E. J. Miller, previously cited, and P. C. Carré et al., J. Clin. Invest., 88, 1882, 1291), glomerulonephritis (T. Wada et al., J. Exp. Med., 180, 1135, 1994) and in the prevention and treatment of damages caused by ischemia and reperfusion.

It is therefore a further object of the present invention to provide compounds for use in the treatment of psoriasis, ulcerative colitis, melanoma, chronic obstructive pulmonary disease (COPD), bullous pemphigo, rheumatoid arthritis, idiopathic fibrosis, glomerulonephritis and in the prevention and treatment of damages caused by ischemia and reperfusion, as well as the use of such compounds in the manufacture of a medicament for the treatment of diseases as described above.

Reduced renal clearance of the compounds of the invention is crucial for the treatment of chronic pathological disorders such as psoriasis and ulcerative cholitis allowing effective therapy with a reduced number of administrations per day up to the optimum once-α-day treatment. Furthermore, the reduced binding of the drugs to the plasmatic proteins mainly albumin is associated to lower effective dosage.

Table I and II show, as an example, the significant differences in T_{1/2} and protein binding (%) between metabolites of formula (I) and corresponding parent compounds of formula (III).

**Table I**

| **Compound** | **Structure Formula** | **T_{1/2} (h)*** | **Protein Binding (%)**** |
|---|---|---|---|
| **Metabolite** | 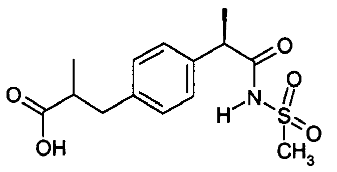 | 2 | 72 |
| (2R) (2" R,S) 2-[4'-(2"-carboxyprop-1-yl) phenyl]propionyl methanesulfonamide | | | |
| **Parent compound** | 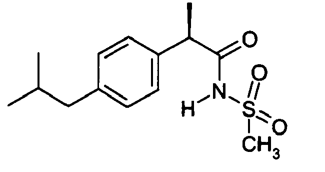 | 0.5 | >99 |
| (R)-2-[(4-isobutyl)phenyl]propionyl methanesulfonamide | | | |

| | | | |
|---|---|---|---|
| *after single bolus i.v. administration in rat (15mg/Kg). ***in vitro* binding to rat plasma proteins (compound concentration 10 µg/mL). | | | |

**Table II**

| **Compound** | **Structure Formula** | **T_{1/2} (h)*** | **Protein Binding (%)**** |
|---|---|---|---|
| **Metabolite** | 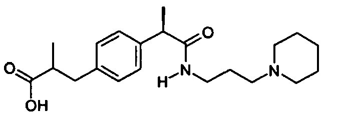 | 2 | 70 |
| (2R)(2"R,S)-[(4'-(2"-carboxypropyl)phenyl]-N-[3"-N'-piperidinopropyl]propionamide | | | |
| **Parent compound** | 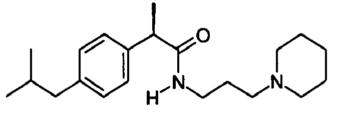 | 1 | 80 |
| (R)-2-[(4-isobutyl)phenyl]-N-[3"-(N'-piperidino)propyl]propionamide | | | |

| | | | |
|---|---|---|---|
| *after single bolus i.v. administration in rat (15mg/Kg). ***in vitro* binding to rat plasma proteins (compound concentration 10 µg/mL). | | | |

Pharmaceutical compositions comprising a compound of the invention and a suitable carrier thereof, are also within the scope of the present invention.

The compounds of the invention, together with a conventionally employed adjuvant, carrier, diluent or excipient may, in fact, be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

When employed as pharmaceuticals, the compounds of this invention are typically administered in the form of a pharmaceutical composition. Such compositions can be prepared in a manner well known in the pharmaceutical art and comprise at least one active compound. Generally, the compounds of this invention are administered in a pharmaceutically effective amount. The amount of the compound actually administered will typically be determined on the basis of relevant circumstances including the condition to be treated, the chosen route of administration, the actual compound administered, the age, weight, and response of the individual patient, the severity of the patient's symptoms, and the like.

The pharmaceutical compositions of the invention can be administered by a variety of routes including oral, rectal, transdermaldermal, subcutaneous, intravenous, intramuscular, and intranasal. Depending on the intended route of delivery, the compounds are preferably formulated as either injectable or oral compositions. The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampoules or syringes of the liquid compositions or pills, tablets, capsules or the like in the case of solid compositions. In such compositions, the acid compound is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors and the like. Liquid forms, including the injectable compositions described herebelow, are always stored in the absence of light, so as to avoid any catalytic effect of light, such as hydroperoxide or peroxide formation. Solid forms may include, for example, any of the following ingredients, or compounds of a similar nature: a binder such as microcrystalline cellulose, gum tragacanth or gelatine; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable carriers known in the art. As above mentioned, the acid derivative of formula (1) in such compositions is typically a minor component, frequently ranging between 0.05 to 10% by weight with the remainder being the injectable carrier and the like. The mean daily dosage will depend upon various factors, such as the seriousness of the disease and the conditions of the patient (age, sex and weight). The dose will generally vary from 1 mg or a few mg up to 1500 mg of the compounds of formula (I) per day, optionally divided into multiple administrations. Higher dosages may be administered also thanks to the low toxicity of the compounds of the invention over long periods of time.

The above described components for orally administered or injectable compositions are merely representative. Further materials as well as processing techniques and the like are set out in Part 8 of "Remington's Pharmaceutical Sciences Handbook", 18th Edition, 1990, Mack Publishing Company, Easton, Pennsylvania, which is incorporated herein by reference.

The compounds of the invention can also be administered in sustained release forms or from sustained release drug delivery systems. A description of representative sustained release materials can also be found in the incorporated materials in the Remington's Handbook as above.

The present invention shall be illustrated by means of the following examples which are not construed to be viewed as limiting the scope of the invention.

Abbreviations: THF: tetrahydrofuran; DMF: dimethylformamide; AcOEt: ethyl acetate.

### EXAMPLES

### Example 1

### (2R) (2" R,S) 2-[4'-(2"-carboxyprop-1-yl)pbenyl]propionyl methanesulfonamide

(R) 2 -(4-bromophenyl)propionic acid (1g, 4,4 mmol) was dissolved in dry CH₂Cl₂ (10 mL). Dimethylaminopyridine (0,45 g, 4,8 mmol), methanesulfonamide (0,46 g, 4,8 mmol) and dicyclohexylcarbodiimide (0,99 g 4.8 mmol) were added and the reaction mixture was left under stirring for 4h. The solution was poured in 1N HCl (10 mL) and the organic phase was separated; the aqueous phase was extracted with dichlomethane (2x 10mL). The collected organic phase was dried over Na₂SO₄. Na₂SO₄ was filtered off and solvent was removed under vacuum to give an oily residue that, after silica gel column purification, provides pure solid (R) 2-(4-bromo-phenyl)propionylmethanesulfonamide in a 70% yield.

(R) 2-(4-bromo-phenyl)propionylmethanesulfonamide was dissolved in tributylamine (2.4 mL) and triphenylphosphine was added . After 5 min. 2-methyl-acrylic acid (0.54 g, 6.64 mmol) and Pd(OAc)₂ (8 mg, 0,033 mmol) were added and the reaction mixture was heated at T= 130°C for 6h. After cooling, the organic solution was poured in 1N HCl and the aqueous phase extracted with EtOAc (3x 15 mL). The collected organic layers were dried over Na₂SO₄ and evaporated at reduced pressure to give an oily residue that, after chromatographic purification, provides a mixture of the two corresponding isomeric unsaturated acids of formula (V) and (VI) in a 30% yield.

The mixture of the two isomers was dissolved in dry MeOH and a catalytic amount of Pd/C 10% (50 mg) was added. The mixture was hydrogenated at r.t. overnight. After removal of the catalyst by filtration on a Celite cake, the filtrate was evaporated and the residue purified on a chromatographic column to give (2R) (2" R,S) 2-[4'-(2"-carboxyprop-1-yl)phenyl]propionyl methanesulfonamide in a 90% yield as a white solid.

¹H-NMR (DMSO): δ 11.95 (bs, 1H, COOH); 11.65 (bs, 1H, SO₂NHCO); 7.18 (m, 4H) ; 3.70 (q, 1H, J=7Hz); 3.18 (s, 3H); 2.85 (m, 1H); 2.58 (m, 2H); 1.32 (d, 3H, J=7Hz); 1.02 (d, 3H, J=7Hz).
[α]_{D}=-12.1 (c=1%, MeOH)

The diastereomeric mixture (2R) (2" R,S) 2-[4'-(2"-carboxyprop-1-yl)phenyl]propionyl methanesulfonamide (10 mg) was purified by semi-preparative HPLC chromatography using a Bondapak C18 125A 15-20 µm (7.8x300mm) column (pH=4.5 phosphate buffer/CH₃CN 90:10 up to 60:40 gradient, run time= 50 min.).
to give the compounds of Examples 1a (2 mg) and 1b (3 mg) as colourless oils.

### Example 1a

(2R) (2" R) 2-[4'-(2"-carboxyprop-1-yl)phenyl]propionyl methanesulfonamide ¹H-NMR (DM SO): δ 11.95 (bs, 1H, COOH); 11.65 (bs, 1H, SO2NHCO 7.18 (m, 4H) ; 3.70 (q, 1H, J=7Hz); 3.18 (s, 3H); 2.85 (m, 1H); 2.58 (d, 2H, J=7Hz); 1.32 (d, 3H, J=7Hz); 1.02 (d, 3H, J=7Hz).

### Example 1b

(2R) (2" S) 2-[4'-(2"-carboxyprop-1-yl)phenyl]propionyl methanesulfonamide ¹H-NMR (DMSO): δ 11.95 (bs, 1H, COOH); 11.65 (bs, 1H, SO2NHCO); 7.18 (m, 4H) ; 3.70 (q, 1H, J=7Hz); 3.18 (s, 3H); 2.85 (m, 1H); 2.60 (d, 2H, J=7Hz); 1.32 (d, 3H, J=7Hz); 1.02 (d, 3H, J=7Hz).

### Example 2

### (2R) (2" R,S) 2-[4'-(2" -carboxyprop-1-yl)phenyl]propionamide

(R) 2 -(4-bromophenyl)propionic acid (1g, 4,4 mmol) was dissolved in dry CH₂Cl₂ (10 mL). Dimethylaminopyridine (0,45 g, 4,8 mmol) and dicyclohexylcarbodiimide (0,99 g 4.8 mmol) were added and ammonia was bubbled in the reaction mixture for 4h. The solution was poured in 1N HCl (10 mL) and the organic phase was separated; the aqueous phase was extracted with dichlomethane (2x 10mL). The collected organic phase was dried over Na₂SO₄ Na₂SO₄ was filtered off and solvent was removed under vacuum to give an oily residue that, after silica gel column purification, provides pure solid (R) 2-(4-bromophenyl)propionamide in a 70% yield.

(R) 2-(4-bromo-phenyl)propionamide (0.70 g, 3.1 mmol) was reacted with 2-methyl-acrylic acid following the same procedure described in Example 1 to give a mixture of the two corresponding isomeric unsaturated acids of Formula (V) and (VI) in a 30% yield.

The mixture was hydrogenated at r.t. overnight. After removal of the catalyst by filtration on a Celite cake, the filtrate was evaporated and the residue purified on a chromatographic column to give (2R) (2" R,S) 2-[4'-(2"-carboxyprop-1-yl)phenyl]propionamide in a 90% yield as a white solid.
¹H-NMR (CDCl3): δ 11.90 (bs, 1H, COOH); 7.20 (d, 2H, J=7Hz); 7.10 (d, 2H, J=7Hz); 5.25 (bs, 2H, CONH2); 3.58 (q, 1H, J=7Hz); 2.70 (m, 1H); 2.42 (m, 2H); 1.18 (d, 3H, J=7Hz); 0.86 (d, 3H, J=7Hz).
[α]_{D}= -26 .1 (c=1%, MeOH)

### Example 3

### (2R)(2"R,S)-[(4'-(2"-carboxypropyl)phenyl]-N-[3"-N',N'-dimethylaminopropyl]propionamide

(2R) (2" R,S) 2-[4'-(2"-carboxyprop-1-yl)phenyl]propionyl methanesulfonamide (0.59 g, 1.8 mmol) was dissolved in CH₂Cl₂; subsequently 3-N,N-dimethylaminoproprylamine (0.37 g, 3,6 mmol) was added and the solution left under stirring at r.t. for 15 min. The solvent was evaporated and the neat solid residue heated at T=120°C under vacuum overnight. The oily residue was cooled and dissolved in water and strongly basic Amberlite resin was added to the mixture. After removal of the water solution, the desired product was recovered by washing the resin with 0.1N HCl. Lyophilisation of the acidic solution afforded (2R)-[(4'-(2"-carboxypropyl]phenyl]-N-[3"-N',N'-dimethylaminopropyl] propionamide (0.35 g, 1.0 mmol, yield= 55%).
¹H-NMR (CDCl3): δ 11.85 (bs, 1H, COOH); 7.10 (s, 4H); 6.00 (bs, 1H, CONH); ( 3.75 (q, 1H, J=7Hz); 3.30 (m, 2H); 2.75 (m, 1H); 2.50 (m, 2H); 2.20 (m, 8H); 1.85 (m, 2H); 1.38 (d, 3H, J=7Hz); 0.90 (d, 3H, J=7Hz).
[α]_{D}= -24 .4 (c=0.5%, MeOH)

### Example 4

### (2R)(2"R,S)-((4'-(2"-carboxypropyl)phenyl]-N-[3"-N'-piperidinopropyl]propionamide

Following the same procedure described in Example 3 using 3-N-piperidinopropylamine (0.5 g, 3.6 mmol) (2R)-[(4'-(2"-carboxypropyl)phenyl]-N-[3"-N'-piperidinopropyl] propionamide (0.3 g, 0.83 mmol, yield= 46%)
¹H-NMR (DMSO): δ 11.95 (bs, 1H, COOH); 7.25 (d, 2H, J=7Hz); 7.12 (d, 2H, J=7Hz); 5.85 (bs, 1H, CONH); 3.58 (q, 1H, J=7 Hz); 3.08 (m, 3H); 2.60 (m, 6H); 2.57 (m, 2H); 1.55 (m, 6H); 1.50 (m, 2H); 1.30 (d, 3H, J=7Hz); 1.02 (d, 3H J=7Hz).
[α]_{D}= -30.4 (c=0.5%, EtOH)

### Example 5

### (2R) (2" R,S) 2-[4'-(2"-carboxyprop-1-yl)phenyl]-N-(4"'-pyridyl)propionamide

Following the same procedure described in Example 3 using 4-aminopyridine (0.34 g, 3.6 mmol) (2R) (2" R,S) 2-[4'-(2"-carboxyprop-1-yl)phenyl]-N-(4"'-pyridyl)propionamide (0.38 g, 1.2 mmol, yield= 67%)
¹H-NMR (CDCl3): δ 11.95 (bs, 1H, COOH); 8.45 (m, 2H); 7.45 (m, 4H); 7.25 (m, 2H); 3.75 (q, 1H, J=7Hz); 3.68 (bs, 1H, CONH); 2.75 (m, 1H); 2.50 (d, 2H, J=7Hz); 1.60 (d, 3H, J=7Hz); 1.00 (d, 3H J=7Hz).
[α]_{D}= -14.4 (c=0.5%, MeOH)

### Example 6

### (2R) (2" R,S) 2-[4'-(2"-carboxyprop-1-yl)phenyl]-N-(2"'-methoxyethyl)propionamide

(2R) (2" R,S) 2-[4'-(2"-carboxyprop-1-yl)phenyl]propionyl methanesulfonamide (0.59 g, 1.8 mmol) was dissolved in CH₂Cl₂; subsequently 2-methoxyethylamine (0.27 g, 3,6 mmol) was added and the solution left under stirring at r.t. for 15 min. The solvent was evaporated and the neat solid residue heated at T=120°C under vacuum overnight. The oily residue was cooled and dissolved in CH₂Cl₂ (20 mL), washed with brine (2x 10 mL). The organic phase was dried over Na₂SO₄. Na₂SO₄ was filtered off and solvent was removed under vacuum to give (2R) (2" R,S) 2-[4'-(2"-carboxyprop-1-yl)phenyl]-N-(2"'-methoxyethyl)propionamide as an oily residue (0.37 g, 1.26 mmol, yield = 70%).
¹H-NMR (CDCl3): δ 11.90 (bs, 1H, COOH); 7.18 (d, 2H, J=7 Hz); 7.08 (d, 2H, J=7Hz); 5.85 (bs, 1H, CONH); 3.60 (q, 1H, J=7 Hz); 3.45 (m, 4H); 3.30 (s, 3H); 2.70 (m, 1H); 2.45 (d, 2H, J= 7Hz); 1.52 (d, 3H J=7Hz); 0.90 (d, 3H, J=7 Hz).
[α]_{D}= -32.4 (c=0.5%, EtOH)

### Example 7

### (2R)(2"R,S)-2-[4'-(2"-carboxyprop-1-yl)phenyll-N-(2"'-carboxymethyl)propionamide

Following the same procedure described in Example 6 using glycine methylester (0.32 g, 3.6 mmol) (2R) (2" R,S) 2-[4'-(2"-carboxyprop-1-yl)phenyl]-N-(2"'-carboxymethyl)propionamide methylester was obtained as an oily residue (0.30 g, 0.97 mmol, yield = 54%). 2-[4'-(2"-carboxyprop-1-yl)phenyl]-N-(2"'-carboxymethyl) propionamide methylester was subsequently dissolved in dioxane (7 mL) and 1N NaOH (1 mL) was added. The solution was left under stirring at r.t. overnight.

Dioxane was evaporated and the aqueous solution was diluted with 5% NaH₂PO₄ buffer (10 mL). The aqueous phase was extracted with CH₂Cl₂ (3x20 mL) and the collected organic phase was dried over Na₂SO₄. Na₂SO₄ was filtered off and solvent was removed under vacuum to give (2R) (2" R,S) 2-[4'-(2"-carboxyprop-1-yl)phenyl]-N-(2"'-carboxymethyl)propionamide as an oily residue (0.23 g, 0.78 mmol, yield = 85%). ¹H-NMR (CDCl3): δ 11.85 (bs, 2H, COOH); 7.20 (d, 2H, J=7 Hz); 7.10 (d, 2H, J=7Hz); 5.95 (bs, 1H, CONH); 4.00 (m, 2H); 3.60 (q, 1H, J=7 Hz); 2.75 (m, 1H); 2.50 (m, 2H); 1.50 (d, 3H J=7Hz); 0.85 (d, 3H, J=7 Hz).
[α]_{D}= -24.0 (c=0.5%, EtOH)
Table III reports chemical name and structure formula for the compounds of Examples 1-7.

**Table III**

| **Example** | **Chemical name** | **Structure Formula** |
|---|---|---|
| **1** | (2R) (2" R,S) 2-[4'-(2"-carboxyprop-1-yl)phenyl] propionyl methanesulfonamide | 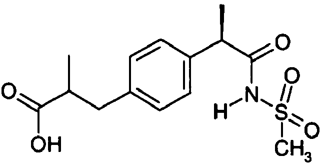 |
| **1a** | (2R) (2" R) 2-[4'-(2"-carboxyprop-1-yl)phenyl] propionyl methanesulfonamide | 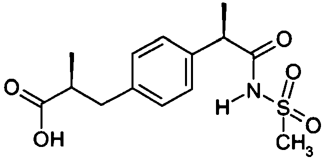 |
| **1b** | (2R) (2" S) 2-[4'-(2"-carboxyprop-1-yl)phenyl] propionyl methanesulfonamide | 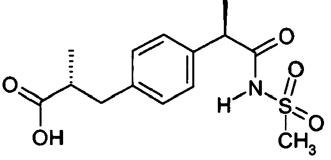 |
| **2** | (2R) (2" R,S) 2-[4'-(2"-carboxyprop-1-yl)phenyl] propionamide | 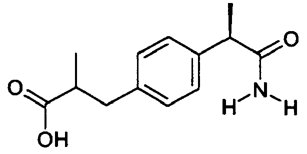 |
| **3** | (2R)(2"R,S)-[(4'-(2"-carboxypropyl)phenyl]-N-[3"-N',N'-dimethylaminopropyl]propionamide | 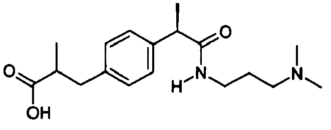 |
| **4** | (2R)(2"R,S)-[(4'-(2"-carboxypropyl)phenyl]-N-[3"-N'-piperidinopropyl]propionamide | 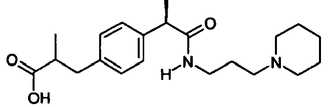 |
| **5** | (2R) (2" R,S) 2-[4'-(2"-carboxyprop-1-yl)phenyl]-N-(4"'-pyridyl)propionamide | 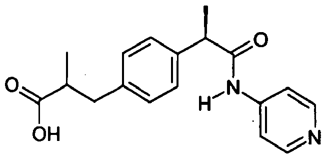 |
| **6** | (2R) (2" R,S) 2-[4'-(2"-carboxyprop-1-yl)phenyl]-N-(2"'-methoxyethyl)propionamide | 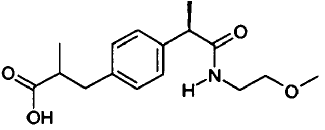 |
| **7** | (2R)(2"R, S)-2-[4'-(2"-carboxyprop-1-yl)phenyl]-N-(2"'-carboxymethyl)propionamide | 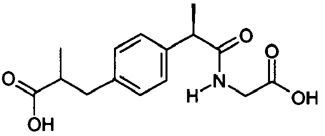 |

## Claims

1. 2-(R)-arylpropionic acid derivative compounds of formula (I) : 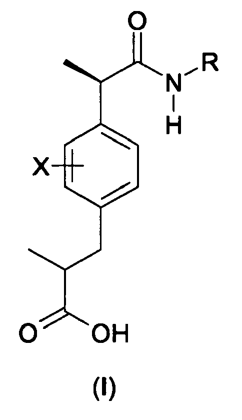 and pharmaceutically acceptable salts thereof,
wherein
X is selected from H, halogen, C₁-C₄-alkyl, C₁-C₄-alkoxy, hydroxy, cyano, nitro, amino;
R group is selected from
- H, OH, C₁-C₅-alkyl, C₁-C₅-cycloalkyl, C₁-C₅-akenyl, C₁-C₅-alkoxy;
- an heteroaryl group selected from pyridine, pyrimidine, pyrrole, tiofene, furane, indole;
- an amino acid residue consisting of straight or branched C₁-C₆-alkyl, C₁-C₆-cydoalkyl, C₁-C₆-alkenyl, C₁-C₆-phenylalkyl, substituted with one further carboxy (COOH) group;
- a residue of formula -CH₂-CH₂-Z-(CH₂-CH₂O)nR' wherein R' is H or C₁-C₅-alkyl, n is an integer from 0 to 2 and Z is oxygen or sulfur;
- a residue of formula -(CH₂)n-NRaRb wherein n is an integer from 0 to 5 and each Ra and Rb, which may be the same or different, are C₁-C₆-alkyl, C₁-C₆-alkenyl or, alternatively, Ra and Rb, together with the nitrogen atom to which they are bound, form a heterocycle from 3 to 7 members of formula (II) 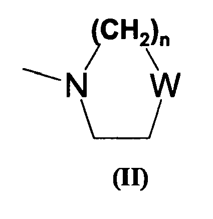 wherein W represents a single bond, O, S, N-Rc, Rc being H, C₁-C₆-alkyl or C₁-C₆-alkylphenyl, and n is an integer from 0 to 4;
- a residue of formula SO₂Rd wherein Rd is C₁-C₆-alkyl, C₁-C₆-cycloalkyl, C₁-C₆-alkenyl.

2. Compounds according to claim 1,
wherein
X group is H;
R group is selected from
H, OH, C₁-C₅ alkyl, C₁-C₅ alkoxy, C₁-C₂-carboxyalkyl;
pyridine, pyrimidine;
a residue of formula -CH₂-CH₂-O-(CH₂-CH₂O)nR' wherein R' is H or C₁-C₅-alkyl, n is the integer 0 or 1;
a residue of formula -(CH₂)n-NRaRb wherein n is the integer 2 or 3, more preferably 3 and the group NRaRb is N,N-dimethylamine, N,N-diethylamine, 1-piperidyl, 4-morpholyl, 1-pyrrolidyl, 1-piperazinyl, 1-(4-methyl)piperazinyl;
a residue of formulaSO₂Rd wherein Rd is C₁-C₂-alkyl;
and single (R) and (S) enantiomers thereof.

3. Compounds according to claims 1 or 2, selected from
(2R) (2" R,S) 2-[4'-(2"-carboxyprop-1-yl)phenyl]propionyl methanesulfonamide
(2R) (2" R,S) 2-[4'-(2"-carboxyprop-1-yl)phenyl]propionamide
(2R) (2" R,S) 2-[4'-(2"-carboxyprop-1-yl)phenyl]-N-(4"'-pyridyl)propionamide
(2R) (2" R,S) 2-[4'-(2"-carboxyprop-1-yl)phenyl]-N-carboxymethyl propionamide
(2R) (2" R,S) 2-[4'-(2"-carboxyprop-1-yl)phenyl]-N-(2"'-methoxyethyl) propionamide
(2R)(2"R,S)2-[4'-(2"-carboxyprop-1-yl)phenyl]-N-[3"-N'-piperidinopropyl] propionamide
(2R)(2"R,S)2-[4'-(2"-carboxyprop-1-yl)phenyl]-N-[3"'-N',N'-dimethylaminopropyl]propionamide
and the single (R) and (S) enantiomers thereof.

4. Process for the preparation of compounds of formula (I) according to claim 1, comprising treatment of the corresponding 2-(4'-aryl)propionamide derivatives of formula (IV), wherein W is Br or OSO₂CF₃ and R is as defined in claim 1, with 2-methylacrylic acid in presence of a suitable catalyst to give a mixture of products of formula (V) and (VI), 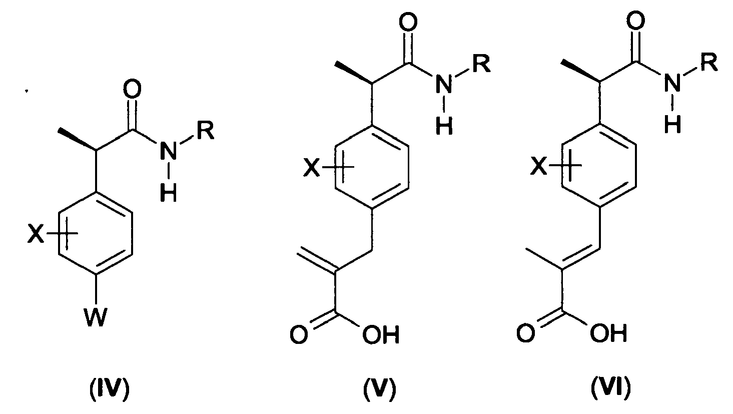 and subsequent hydrogenation in the presence of a suitable catalyst.

5. Process for the preparation of compounds of formula (I) according to claim 1, wherein R is a group as defined in claim 1, but is not OH or C₁-C₅-alkoxy or a residue SO₂Rd, comprising treatment of an acylmethansulfonamide derivative of formula (I) according to claim 1, wherein R is SO₂Rd, with two equivalents of a suitable amine of formula NH₂R, and heating the salt thereby obtained at a temperature of about 100-140°C.

6. Compounds according to claim 1 for use as medicaments.

7. Compounds according to claim 6 for the inhibition of the chemotaxis of polymorphonucleate and mononucleate cells.

8. Use of compounds of formula (I) according to claim 1 in the manufacture of a medicament for the treatment of psoriasis, ulcerative colitis, melanoma, chronic obstructive pulmonary disease (COPD), bullous pemphigo, rheumatoid arthritis, idiopathic fibrosis, glomerulonephritis and in the prevention and treatment of damages caused by ischemia and reperfusion.

9. Pharmaceutical compositions comprising a compound of formula (I) according to claim 1 and a suitable carrier thereof.

## Patentansprüche

1. Von 2-(R)-Arylpropionsäure abgeleitete Verbindungen der Formel (I) : 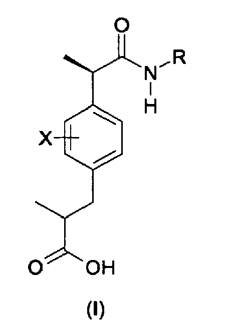 sowie pharmazeutisch akzeptable Salze derselben,
wobei
X aus H, Halogen, C₁-C₄-Alkyl, C₁-C₄-Alkoxy, Hydroxy, Cyano, Nitro, Amino; ausgewählt ist,
die R-Gruppe aus
- H, OH, C₁-C₅-Alkyl, C₁-C₅-Cycloalkyl, C₁-C₅-Alkenyl, C₁ - C₅-Alkoxy;
- einer aus Pyridin, Pyrimidin, Pyrrol, Tiofen, Furan, Indol ausgewählten Heteroarylgruppe;
- einem Rest von Amminosäure, der aus durch eine weitere Carboxygruppe (COOH) substituirtem, geradekettigem bzw. verzweigtem C₁-C₆-Alkyl, C₁-C₆-Cycloalkyl, C₁-C₆-Alkenyl, C₁-C₆-Phenylalkyl besteht;
- einem Rest der Formel -CH₂-CH₂-Z-(CH₂ ,-CH₂O)nR', wobei R' für H oder gleich C₁-C₅-Alkyl steht, n eine ganze Zahl von 0 bis 2 ist, und Z ein Sauerstoff oder Schwefel ist;
- einem Rest der Formel -(CH₂)n-NRaRb, wobei n eine ganze Zahl von 0 bis 5 ist und je Ra als auch Rb, die gleich oder verschieden sein können, C₁-C₆-Alkyl, C₁-C₆-Alkenyl sind, abwechselnd Ra und Rb zusammen mit dem Stickstoffatom, an welches sie gebunden sind, einen Heterozyklus von 3 bis 7 Gliedern der Formel (II) 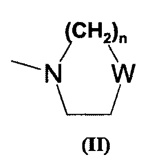 bilden,
wobei W für eine Einzelbindung, O, S, N-Rc steht, wo Rc gleich H, C₁-C₆-Alkyl oder C₁-C₆-Alkylphenyl ist und n eine ganze Zahl von 0 bis 4 ist;
- einem Rest der Formel SO₂Rd wobei Rd gleich C₁-C₆-Alkyl, C₁-C₆-Cykloalkyl, C₁-C₆-Alkenyl ist,
ausgewählt ist.

2. Verbindungen nach Anspruch 1,
wobei
die X-Gruppe gleich H ist;
die R-Gruppe aus
H, OH, C₁-C₅-Alkyl, C₁-C₅-Alkoxy, C₁-C₂-Carboxyalkyl;
Pyridin, Pyrimidin;
einem Rest der Formel -CH₂-CH₂-O-(CH₂-CH₂O)nR', wobei R' gleich H oder C₁-C₅-Alkyl ist, n die ganze Zahl 0 oder 1 ist;
einem Rest der Formel -(CH₂)n-NRaRb, wobei n die ganze Zahl 2 oder 3, vorzugsweise 3, ist, und die Gruppe NRaRb gleich N,N-Dimethylamin, N,N-Diethylamin, 1-Piperidyl, 4-Morpholyl, 1-Pyrrolidyl, 1-Piperazinyl, 1-(4-Methyl)piperazinyl ist;
einem Rest der Formel SO₂Rd wobei Rd gleich C₁-C₂-Alkyl ist,
ausgewählt sind,
sowie einzelne Enantiomeren (R) und (S) derselben;

3. Verbindungen nach Anspruch 1 oder 2; aus
(2R) (2"R,S) 2-[4'-(2"-Carboxyprop-1-yl)phenyl]propionyl methansulfonamid
(2R) (2"R,S) 2-[4'-(2"-Carboxyprop-1-yl)phenyl]propionamid
(2R) (2"R,S) 2-[4'-(2"-Carboxyprop-1-yl)phenyl]-N-(4"'-Pyridyl)propionamid
(2R) (2"R,S) 2-[4'-(2"-Carboxyprop-1-yl)phenyl]-N-carboxymethyl propionamid
(2R)(2"R,S)2-[4'-(2"-Carboxyprop-1-yl)phenyl]-N-(2"'-methoxyethyl) propionamid
(2 R)(2"R, S )2-[4'-(2"-Ca rboxyprop-1-yl )ph enyl]-N-[3"-N'-piperidinopropyl]propionamid
(2 R)(2"R,S)2-[4'-(2"-Carboxyprop-1-yl)phenyl]-N-[3"'-N',N'-dimethylaminopropyl]propionamid ausgewählt;
sowie die einzelnen Enantiomeren (R) und (S) derselben.

4. Verfahren für die Herstellung von Verbindungen der Formel (I) nach Anspruch 1, welches die Behandlung der entsprechenden 2-(4'-Aryl)propionamid-Derivate von Formel (IV), wobei W gleich Br oder gleich OSO₂CF₃ ist, und R gemäß der Bestimmung in Anspruch 1 ist, mit 2-Methylacrylsäure in Gegenwart von einem geeigneten Katalysator um ein Gemisch der Produkte der Formel (V) und (VI) zu verwirklichen, 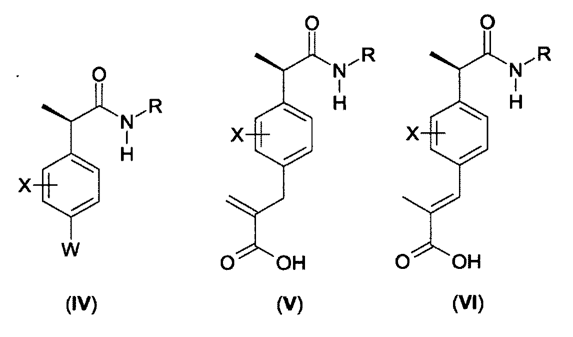 sowie nachfolgende Hydrogenierung in Gegenwart von einem geeigneten Katalysators umfasst.

5. Verfahren für die Herstellung von Verbindungen der Formel (I) nach Anspruch 1, wobei R eine wie in Anspruch 1 bestimmte Gruppe ist aber nicht OH oder C₁-C₅-Alkoxy oder ein Rest von SO₂R ist, welches eine Behandlung eines Acyl-methansulfonamid-Derivats der Formel (I) nach Anspruch 1, wobei R gleich SO₂R ist, mit zwei Äquivalenten eines geeigneten Amins der Formel NH₂R, und Heizung des **dadurch** erhaltenen Salzes auf eine Temperatur um 100° - 140°C umfasst,.

6. Verbindungen nach Anspruch 1 zur Verwendung als Medikamente.

7. Verbindungen nach Anspruch 6 zur Hemmung der Chemotaxis von polymorphonuklearen und mononuklearen Zellen.

8. Verwendung der Verbindungen der Formel (I) nach Anspruch 1 bei der Herstellung eines Medikaments für die Behandlung von Psoriasis, Colitis ulcerosa, Melanom, chronisch obstruktiver Lungenerkrankung (COPD), Pemphigus, chronischem Gelenkrheumatismus, idiopathischer Fibrose, Glomerulonephritis sowie für die Vorbeugung und Behandlung von durch Ischämie und Reperfusion verursachten Schädigungen.

9. Pharmazeutische Zusammensetzung, die eine Verbindung der Formel (I) nach Anspruch 1 sowie einen Träger derselben umfasst.

## Revendications

1. Composés de dérivés de l'acide 2-(R)-arylpropionique de formule (I) : 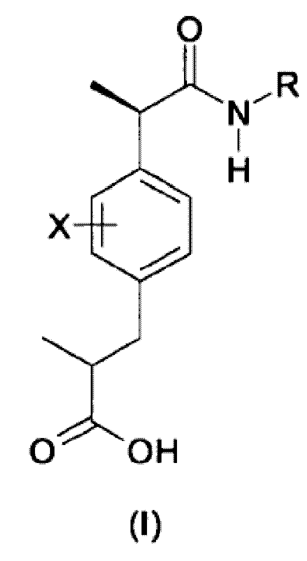 et leurs sels pharmaceutiquement acceptables,
où
X est choisie parmi H, halogène, C₁-C₄-alkyle, C₁-C₄-alkoxy, hydroxy, cyano, nitro, amino ;
le groupement R est choisie parmi
- H, OH, C₁-C₅-alkyle, C₁-C₅-cycloalkyle, C₁-C₅-alcényle, C₁-C₅-alkoxy ;
- un groupement hétéroaryle choisi parmi pyridine, pyrimidine, pyrrole, thiofène, furanne, indole ;
- un résidu d'acide aminé constitué de C₁-C₆-alkyle, C₁-C₆-cycloalkyle, C₁-C₆-alcényle, C₁-C₆-phénylalkyle, linéaire ou ramifié, substitué par un autre groupement carboxy(COOH);
- un résidu de formule -CH₂-CH₂-Z-(CH₂-CH₂O)R' où R' est H ou C₁-C₅-alkyle, n est un entier de 0 à 2 et Z est oxygène o soufre ;
- un résidu de formule -(CH₂)n-NRaRb où n est un entier de 0 à 5 et Ra et Rb, qui peuvent être identiques ou différents, sont chacun C₁-C₆-alkyle, C₁-C₆-alcényle ou, alternativement, Ra et Rb, forment avec l'atome d'azote auquel ils sont liés, un hétérocycle à 3-7 chaînons de formule (II) 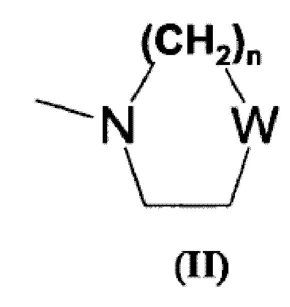 où W représente une liaison simple, O, S, N-Rc, Rc étant H, C₁-C₆-alkyle ou C₁-C₆-alkylphényle, et n est un entier de 0 à 4 ;
- un résidu de formule SO₂R où Rd est C₁-C₆-alkyle, C₁-C₆-cycloalkyle, C₁-C₆-alcényle.

2. Composés selon la revendication 1,
où
le groupement X est H ;
le groupement R est choisie parmi H, OH, C₁-C₅ alkyle, C₁-C₅ alkoxy, C₁-C₂-carboxyalkyle ;
pyridine, pyrimidine ;
un résidu de formule -CH₂-CH₂-O-(CH₂-CH₂O)ₙR' où R' est H ou C₁-C₅-alkyle, n est l'entier 0 ou 1 ;
un résidu de formule -(CH₂)ₙ-NRaRb où n est l'entier 2 ou 3, plus préférentiellement 3 et le groupement NRaRb est N,N-diméthylamine, N,N-diéthylamine, 1-pipéridyle, 4-morpholyle, 1-pyrrolidyle, 1-pipérazinyle, 1-(4-méthyl)pipérazinyle ;
un résidu de formule SO₂R où Rd est C₁-C₂-alkyle ;
et leurs énantiomères individuelles (R) et (S).

3. Composés selon les revendications 1 ou 2, choisis parmi
(2R)(2"R,S) 2-[4'-(2"-carboxyprop-1-yl)phényl]propionyl méthanesulfonamide,
(2R)(2"R,S) 2-[4'-(2"-carboxyprop-I-yl)phényl]propionamide,
(2R) (2"R,S) 2-[4'-(2"-carboxyprop-1-yl)phényl]-N-(4"'-pyridyl)propionamide,
(2R) (2"R,S) 2-[4'-(2"-carboxyprop-1-yl)phényl]-N-carboxymethyl propionamide,
(2R) (2"R,S) 2-[4'-(2"-carboxyprop-1-yl)phényl]-N-(2"'-méthoxyéthyl) propionamide,
(2R)(2"R,S)2-[4'-(2"-carboxyprop-I-yl)phényl]-N-[3"-N'-pipéridinopropyl] propionamide,
(2 R)(2"R,S)2-[4'-(2"-carboxyprop-1-yl)phényl]-N-[3"'-N',N'-diméthylaminopropyl]propionamide,
et leurs énantiomères individuelles (R) et (S).

4. Procédé de préparation de composés de formule (I) selon la revendication 1, comprenant le traitement des dérivés de 2-(4'-aryl)propionamide correspondants de formule (IV), où W est Br ou OSO₂CF₃ et R est tel que défini à la revendication 1, avec de l'acide 2- méthylacrylique en présence d'un catalyseur approprié donnant un mélange de produits de formule (V) et (VI ), 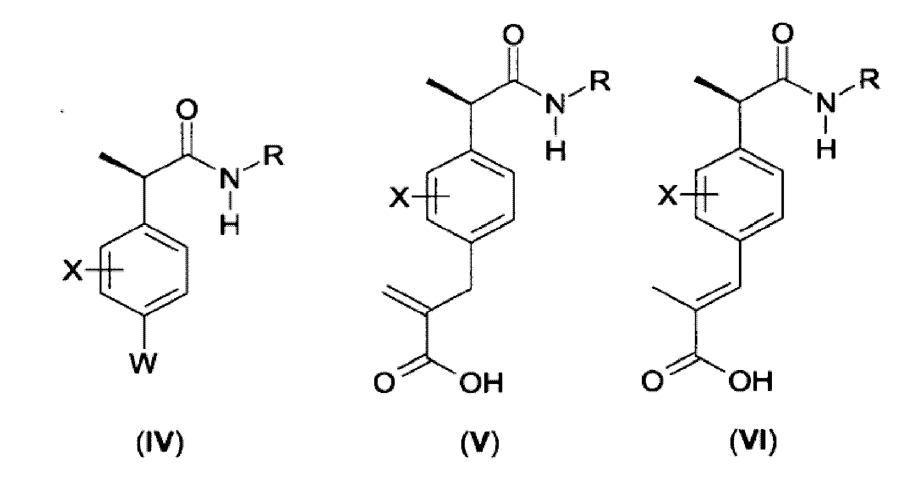 suivi de l'hydrogénation en présence d'un catalyseur approprié.

5. Procédé de préparation de composés de formule (I) selon la revendication 1, où R est un groupement tel que défini à la revendication 1, R n'est pas OH ou C₁-C₅-alkoxy ou un résidu SO₂Rd comprenant le traitement d'un dérivé de l'acylméthanesulfonamide de formule (I) selon la revendication 1, où R est SO₂Rd avec deux équivalents d'une amine appropriée de formule NH₂R, et chauffage du sel obtenu de cette façon à une température d'environ 100-140°C.

6. Composés selon la revendication 1 pour l'utilisation comme médicaments.

7. Composés selon la revendication 6 pour l'inhibition de la chimiotaxie des cellules polymorphonucléaires et mononucléaires.

8. Utilisation des composés de formule (I) selon la revendication 1 dans la préparation d'un médicament pour le traitement de psoriasis, recto-colite hémorragique, mélanome, bronchopneumopathie chronique obstructive (COPD), pemphigoïde bulleuse, arthrite rhumatoïde, fibrose idiopathique, glomérulonéphrite et dans la prévention et le traitement des dommages causés par l'ischémie et la reperfusion.

9. Compositions pharmaceutiques comprenant un composé de formule (1) selon la revendication 1 et un vecteur approprié pour celui-ci.
